# EUROPEAN PATENT APPLICATION

(11) **EP 4 715 211 A1**
(43) Date of publication of application: **25.03.2026**
(21) Application number: 24811039.7
(22) Date of filing: 16.05.2024
(51) Int. Cl.: F04B 43/02, A61M 1/16

(54) **PUMP DEVICE**

(30) Priority: 19.05.2023 JP 2023083477
(71) Applicant: Nikkiso Company Limited, Tokyo 150-6022 (JP)
(72) Inventor: MOCHIZUKI Hiroaki, Higashimurayama-shi, Tokyo 189-8520 (JP); TOKUNAGA Makoto, Higashimurayama-shi, Tokyo 189-8520 (JP)
(74) Representative: von Hirschhausen, Helge
(86) International application number: PCT/JP2024/018242
(87) International publication number: WO 2024/242032

(57) **Abstract**

Provided is a pump device that includes a chamber sealed in an easy and assured manner, that eliminates the necessity for additional pumps, and that is capable of controlling the flow rate of liquid with high accuracy. A pump device 1 includes a chamber 2 capable of accommodating a predetermined volume of liquid; a dividing membrane 5 made of a flexible member attached to the chamber 2, the dividing membrane 5 dividing an inside of the chamber 2 into a first area 3 and a second area 4, the dividing membrane 5 being displaced alternately into the first area 3 and into the second area 4 to cause the liquid to be suctioned into and discharged from the first area 3 and cause drain liquid to be suctioned into and discharged from the second area 4; and a core member 6 provided to the dividing membrane 5 and configured to undergo a reciprocating motion inside the chamber 2 with activation of a motor M and in such a manner as to displace the dividing membrane 5 into the first area 3 and into the second area 4.

## Description

### Technical Field

The present invention relates to a pump device configured to suction and discharge liquid into and from a first area and suction and discharge drain liquid into and from a second area.

### Background Art

A blood purifier, such as a dialyzer, intended for hemodialysis treatment is provided with a dialysate supply line for supplying dialysate and a dialysate drain line for discharging drain dialysate. The dialysate supply line and the dialysate drain line extend from a dialysis device and are connected to the blood purifier, thereby enabling the dialysate to be supplied to the blood purifier and simultaneously enabling the drain dialysate to be discharged from the blood purifier.

The dialysis device includes a pump device provided astride the dialysate supply line and the dialysate drain line. One existing pump device (first existing example) is a reciprocating metering pump including a cylinder constituted by a pair of pump chambers provided with respective pistons, as disclosed in PTL 1 for example. In such a reciprocating metering pump, an electric motor or the like activates a crank mechanism to cause the pistons to undergo a reciprocating motion inside the cylinders, whereby the delivery of dialysate to a dialyzer is enabled.

Another existing pump device (second existing example) includes, as disclosed in PTL 2 for example, a chamber divided by an elastic member, such as a diaphragm, into a first area and a second area; and electromagnetic valves provided to respective flow paths connected to the first area and the second area. In such a pump device, the electromagnetic valves are controlled to open and close the respective flow paths with arbitrary timings, whereby the delivery of dialysate to a dialyzer is enabled.

### Citation List

### Patent Literature

PTL 1: Japanese Unexamined Patent Application Publication No. S55-167009
PTL 2: Japanese Unexamined Patent Application Publication No. S53-142382

### Summary of Invention

### Technical Problem

In the pump device according to the first existing example, although a sealing part is provided between each of the pistons and the cylinder, liquid leakage may occur at the sealing part while the piston is undergoing a reciprocating motion in the cylinder. In the pump device according to the second existing example, an additional pump for liquid delivery to the chamber is necessary.

The present invention has been conceived in view of the above circumstances and provides a pump device that includes a chamber sealed in an easy and assured manner, that eliminates the necessity for additional pumps, and that is capable of controlling the flow rate of liquid with high accuracy.

### Solution to Problem

A pump device according to an embodiment of the present invention includes a chamber capable of accommodating a predetermined volume of liquid; a dividing membrane made of a flexible member attached to the chamber, the dividing membrane dividing an inside of the chamber into a first area and a second area; and a core member provided to the dividing membrane and configured to undergo a reciprocating motion inside the chamber with activation of a drive source and in such a manner as to displace the dividing membrane into the first area and into the second area. Displacing the dividing membrane alternately into the first area and into the second area causes the liquid to be suctioned into and discharged from the first area and causes drain liquid to be suctioned into and discharged from the second area. Advantageous Effects of Invention

The present invention employs the dividing membrane that is displaced alternately into the first area and into the second area to cause the liquid to be suctioned into and discharged from the first area and cause the drain liquid to be suctioned into and discharged from the second area, and the core member provided to the dividing membrane and configured to undergo a reciprocating motion inside the chamber with the activation of the drive source and in such a manner as to displace the dividing membrane into the first area and into the second area. Thus, the chamber is sealed in an easy and assured manner, the necessity for additional pumps is eliminated, and the flow rate of the liquid is controllable with high accuracy.

### Brief Description of Drawings

[Fig. 1] Fig. 1 is an external front perspective view of a pump device according to an embodiment of the present invention.
[Fig. 2] Fig. 2 is an external rear perspective view of the pump device.
[Fig. 3] Fig. 3 is a three-view drawing of the pump device.
[Fig. 4] Fig. 4 illustrates a section taken along line IV-IV given in Fig. 3, with a dividing membrane being at the center position.
[Fig. 5] Fig. 5 illustrates the section taken along line IV-IV given in Fig. 3, with the dividing membrane displaced into a second area.
[Fig. 6] Fig. 6 illustrates the section taken along line IV-IV given in Fig. 3, with the dividing membrane displaced into a first area.
[Fig. 7] Fig. 7 illustrates a section taken along line V-V given in Fig. 3.
[Fig. 8] Fig. 8 is an exploded perspective view of major components included in the pump device.
[Fig. 9] Fig. 9 is a perspective view of the dividing membrane included in the pump device and with which a core member is integrated.
[Fig. 10] Fig. 10 is a perspective view of the core member included in the pump device.
[Fig. 11] Fig. 11 is a perspective view of a motor and elements therearound included in the pump device.
[Fig. 12] Fig. 12 is a perspective view of the motor and a cam member included in the pump device.
[Fig. 13] Fig. 13 is a perspective view of a cam-receiving member and elements therearound included in the pump device.
[Fig. 14] Fig. 14 is a schematic diagram illustrating the piping (flow paths for dialysate and drain liquid) of a hemodialysis apparatus to which the pump device is connected.
[Fig. 15] Fig. 15 is a schematic diagram illustrating the piping of a hemodialysis apparatus to which a pump device according to another embodiment of the present invention is connected.
[Fig. 16] Fig. 16 includes graphs illustrating how dialysate is supplied from the pump device to a blood purifier.
[Fig. 17] Fig. 17 is a schematic diagram illustrating the piping of a hemodialysis apparatus to which a pump device according to yet another embodiment of the present invention is connected.

### Description of Embodiments

Embodiments of the present invention will now be described specifically with reference to the drawings.

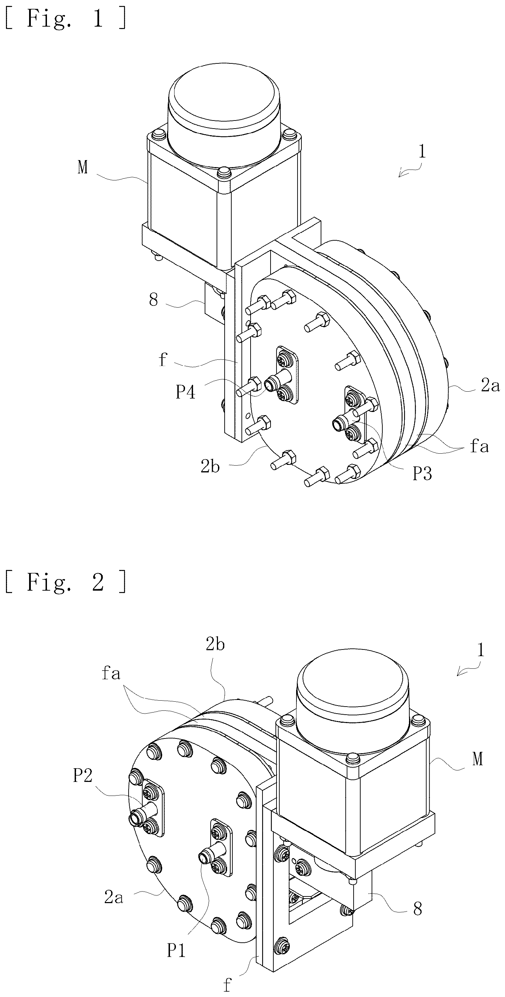

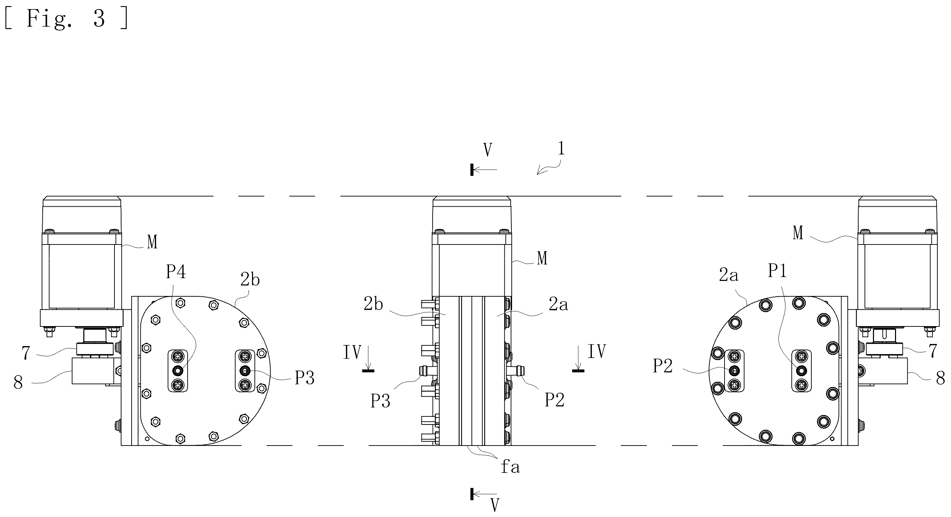

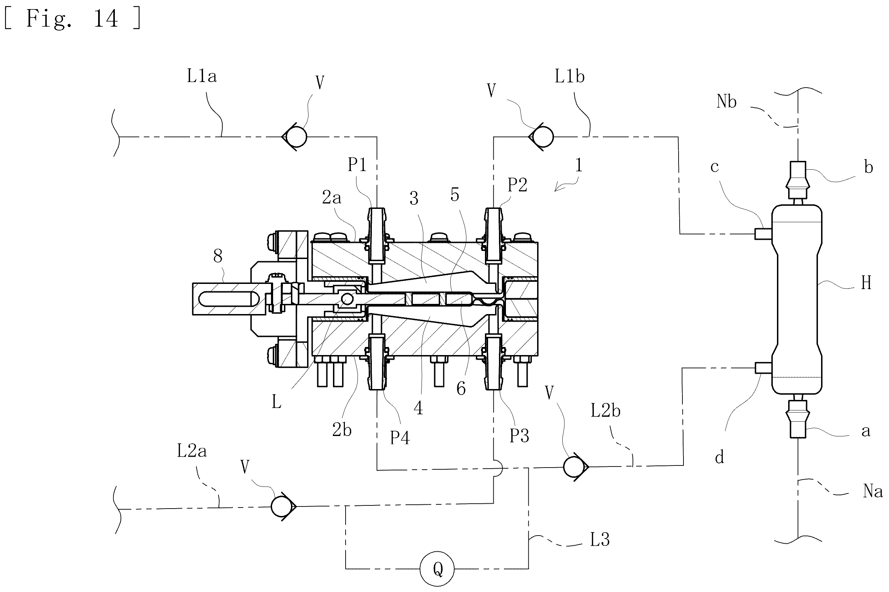

A pump device 1 according to the present embodiment is applied to a hemodialysis apparatus as illustrated in Fig. 14 and is configured to supply dialysate to a blood purifier H and simultaneously discharge drain liquid from the blood purifier H. As illustrated in Figs. 1 to 8, the pump device 1 includes a chamber 2, which includes a first chamber 2a and a second chamber 2b; a dividing membrane 5, which is provided inside the chamber 2; a core member 6, which is provided to the dividing membrane 5; and a motor M, which serves as a drive source.

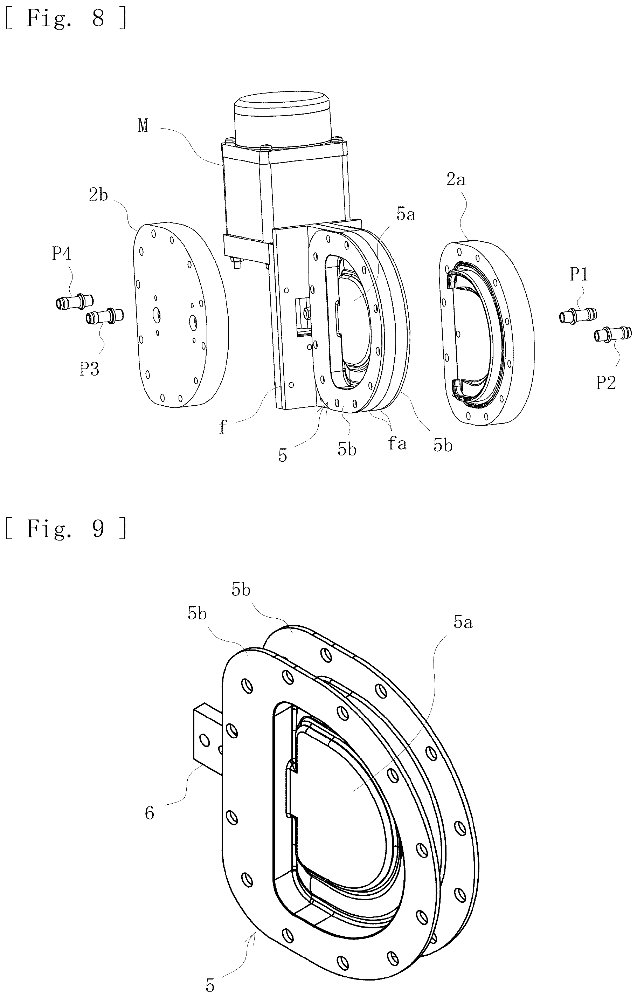

As illustrated in Fig. 8, the chamber 2 has thereinside an accommodation space obtained by mating the first chamber 2a and the second chamber 2b with each other from the left and right. The accommodation space is capable of accommodating a predetermined volume of liquid (dialysate, in the present embodiment). The pump device 1 according to the present embodiment includes a frame f, which includes a pair of frame parts fa. The first chamber 2a and the second chamber 2b are attached to the respective frame parts fa from the left and right, whereby the accommodation space is obtained. The first chamber 2a and the second chamber 2b according to the present embodiment are fastened to each other with a plurality of bolts, as illustrated in the drawings.

First connection ports (P1 and P2) project from the first chamber 2a. Second connection ports (P3 and P4) project from the second chamber 2b. As illustrated in Fig. 14, the first connection ports (P1 and P2) and the second connection ports (P3 and P4) according to the present embodiment are projecting members that are connectable to dialysate introduction lines (L1a and L1b), through which dialysate is introduced into the blood purifier H, and dialysate drain lines (L2a and L2b), through which drain liquid is discharged from the blood purifier H.

Specifically, the first connection port P1 is connected to the dialysate introduction line L1a, which is connected to a dialysate supply source (not illustrated), whereas the first connection port P2 is connected to the dialysate introduction line L1b, which is connected to a dialysate introduction port c of the blood purifier H. Furthermore, the second connection port P3 is connected to the dialysate drain line L2a, which is connected to draining means (not illustrated), whereas the second connection port P4 is connected to a dialysate drain port d of the blood purifier H.

The dialysate introduction lines (L1a and L1b) and the dialysate drain lines (L2a and L2b) are provided with respective check valves V, whereby the flow direction of the dialysate is restricted. Specifically, the check valve V provided to the dialysate introduction line L1a allows the flow of the dialysate in the dialysate introduction line L1a from the supply source toward the pump device 1 while restricting the reverse flow. The check valve V provided to the dialysate introduction line L1b allows the flow of the dialysate in the dialysate introduction line L1b from the pump device 1 toward the blood purifier H while restricting the reverse flow.

The check valve V provided to the dialysate drain line L2b allows the flow of the dialysate in the dialysate drain line L2b from the blood purifier H toward the pump device 1 while restricting the reverse flow. The check valve V provided to the dialysate drain line L2a allows the flow of the dialysate in the dialysate drain line L2a from the pump device 1 toward the dialysate draining means while restricting the reverse flow. The check valves V may be replaced with electromagnetic valves. In that case, increased sealability is achieved.

The blood purifier H applied to the present embodiment has a blood introduction port a, which is connectable to an arterial blood circuit Na through which a patient's blood is made to extracorporeally circulate; a blood delivery port b, which is connectable to a venous blood circuit Nb; the dialysate introduction port c, which is connectable to the dialysate introduction line L1b; and the dialysate drain port d, which is connectable to the dialysate drain line L2b. The blood purifier H includes inside the housing thereof a plurality of hollow fiber membranes (blood purification membranes).

The hollow fiber membranes included in the blood purifier H each have a plurality of microscopic holes. Therefore, when dialysate is made to flow outside the hollow fibers while blood is made to flow inside the hollow fibers, unnecessary matter (waste products) in the blood are allowed to permeate through the hollow fiber membranes to the dialysate and are thus removed. Between the dialysate drain line L2a and the dialysate drain line L2b is connected a bypass line L3, which is provided with an ultrafiltration pump Q. Activating the ultrafiltration pump Q enables ultrafiltration, in which water is removed from the patient's blood flowing through the blood purifier H.

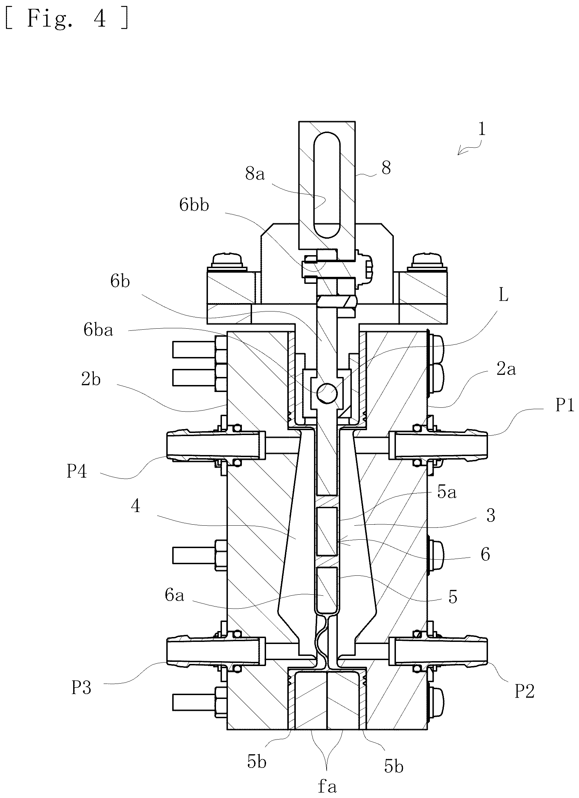

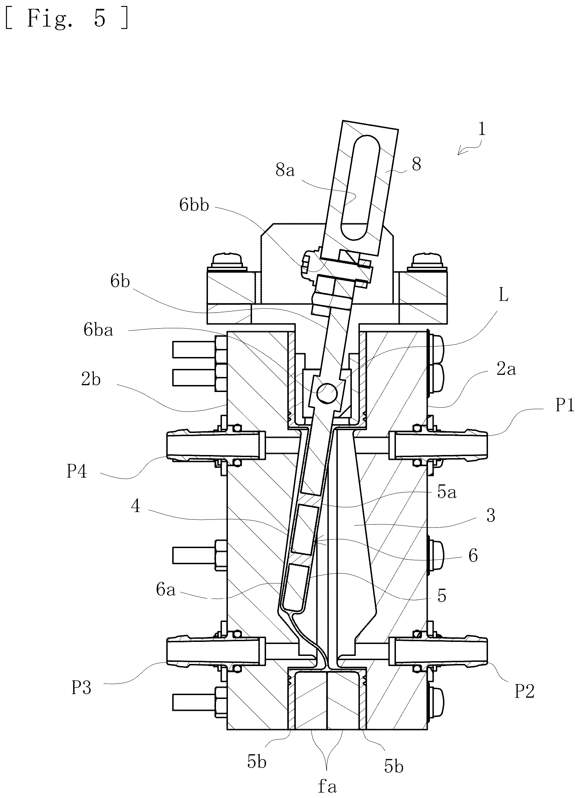

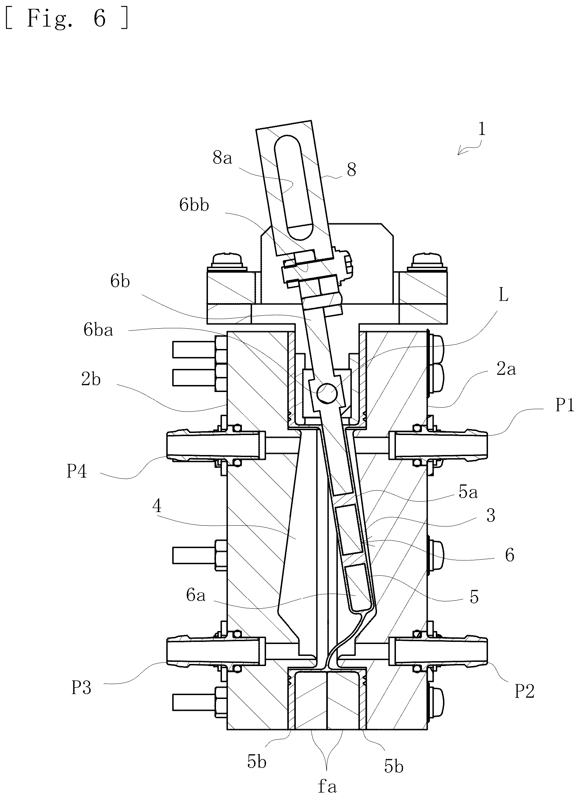

The dividing membrane 5 is made of a flexible member (for example, a rubber material, a resin material, or the like) provided inside (in the accommodation space of) the chamber 2. As illustrated in Figs. 4 to 8, the dividing membrane 5 divides the inside of the chamber 2 into a first area 3 and a second area 4. The dividing membrane 5 is displaced alternately into the first area 3 and into the second area 4, which causes the dialysate to be suctioned into and discharged from the first area 3 and causes the drain liquid to be suctioned into and discharged from the second area 4. Specifically, the dividing membrane 5 swings into the first area 3 as illustrated in Fig. 6 and swings into the second area 4 as illustrated in Fig. 5 to cause the dialysate to be suctioned into and discharged from the first area 3 and cause the drain liquid to be suctioned into and discharged from the second area 4.

The first area 3 communicates with the dialysate introduction lines (L1a and L1b) through the first connection ports (P1 and P2). The second area 4 communicates with the dialysate drain lines (L2a and L2b) through the second connection ports (P3 and P4). The dividing membrane 5 according to the present embodiment is made of a flexible member and is displaceable while dividing the inside of the chamber 2. That is, the dividing membrane 5 serves as a diaphragm and is capable of causing the dialysate to be suctioned into and discharged from the first area 3 and causing the drain liquid to be suctioned into and discharged from the second area 4.

Specifically, when the dividing membrane 5 is displaced into the first area 3 (when the dividing membrane 5 swings as illustrated in Fig. 6), the dialysate in the first area 3 is discharged through the dialysate introduction line L1b into the blood purifier H, whereas the drain liquid in the blood purifier H is suctioned through the dialysate drain line L2b into the second area 4. On the other hand, when the dividing membrane 5 is displaced into the second area 4 (when the dividing membrane 5 swings as illustrated in Fig. 5), the dialysate in the second area 4 is discharged through the dialysate drain line L2a into the draining means, whereas the dialysate supplied from the supply source is suctioned through the dialysate introduction line L1a into the first area 3.

As illustrated in Fig. 9, the dividing membrane 5 according to the present embodiment includes a dividing portion 5a and sealing portions 5b, which are formed integrally with each another. The dividing portion 5a separates the first area 3 and the second area 4 from each other. The sealing portions 5b seal the first area 3 and the second area 4. Specifically, with the dividing membrane 5 set inside the chamber 2, the dividing portion 5a, which forms a central portion, is located inside the accommodation space of the chamber 2 and separates the first area 3 and the second area 4 from each other, and the sealing portions 5b, which form peripheral portions, are each located between a corresponding one of the first chamber 2a and the second chamber 2b and a corresponding one of the frame parts fa and thus seal the first chamber 2a and the second chamber 2b.

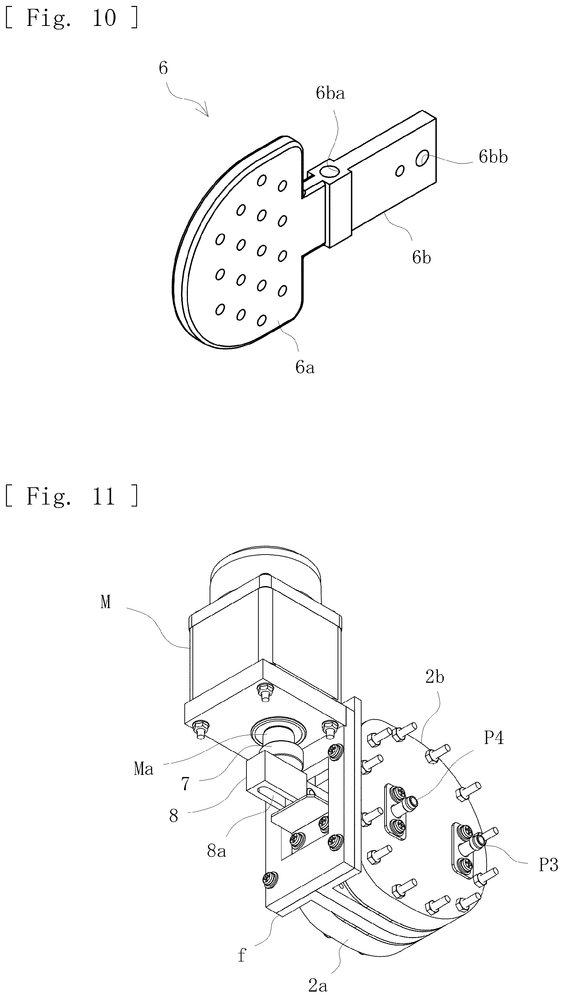

The core member 6 is provided to the dividing membrane 5 and is configured to undergo a reciprocating motion inside the chamber 2 with the activation of the motor M (drive source) and in such a manner as to displace the dividing membrane 5 into the first area 3 and into the second area 4. As illustrated in Fig. 10, the core member 6 includes a distal portion 6a, which has a large width; and an extended portion 6b, which is extended from the distal portion 6a. The core member 6 is a metal member or is made of a rigid resin material or the like. The distal portion 6a has a plurality of holes or recesses. The extended portion 6b has an insertion hole 6ba, through which a shaft member L (see Figs. 4 and 5) is inserted; and a screw hole 6bb, through which a screw for the coupling to a cam-receiving member 8 is inserted.

As illustrated in Fig. 9, the core member 6 according to the present embodiment has a distal portion 6a that is integrated into the dividing membrane 5, with the extended portion 6b projecting from the dividing membrane 5. Thus, the distal portion 6a located inside the chamber 2 is kept out of contact with the dialysate in the first area 3 and the drain liquid in the second area 4. Specifically, the core member 6 is molded together with the dividing membrane 5 through insert molding. Thus, the distal portion 6a is firmly fixed to the dividing membrane 5 to be integrated therewith. As illustrated in Fig. 4, the core member 6 is coupled to the cam-receiving member 8, which has a groove 8a, with a screw inserted into the screw hole 6bb of the extended portion 6b.

The core member 6 is set in the pump device 1 with the shaft member L inserted into the insertion hole 6ba of the extended portion 6b. A driving force generated by the motor M causes the core member 6 to swing about the shaft member L in such a manner as to undergo a reciprocating motion inside the chamber 2. Thus, the dividing membrane 5 is displaceable inside the chamber 2. Hence, as illustrated in Fig. 4, the chamber 2 according to the present embodiment has a fan-shaped accommodation space spreading along the locus of swing of the core member 6 (a fan-shaped space centered on the shaft member L).

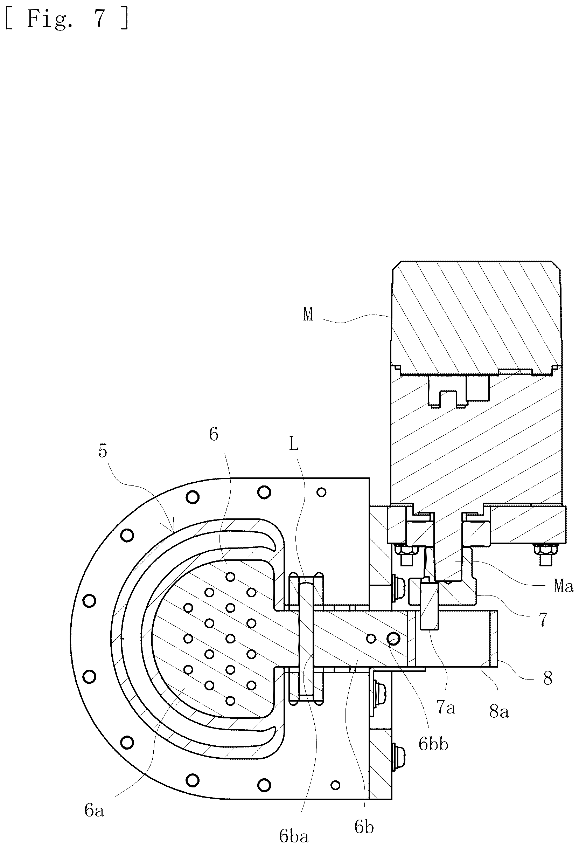

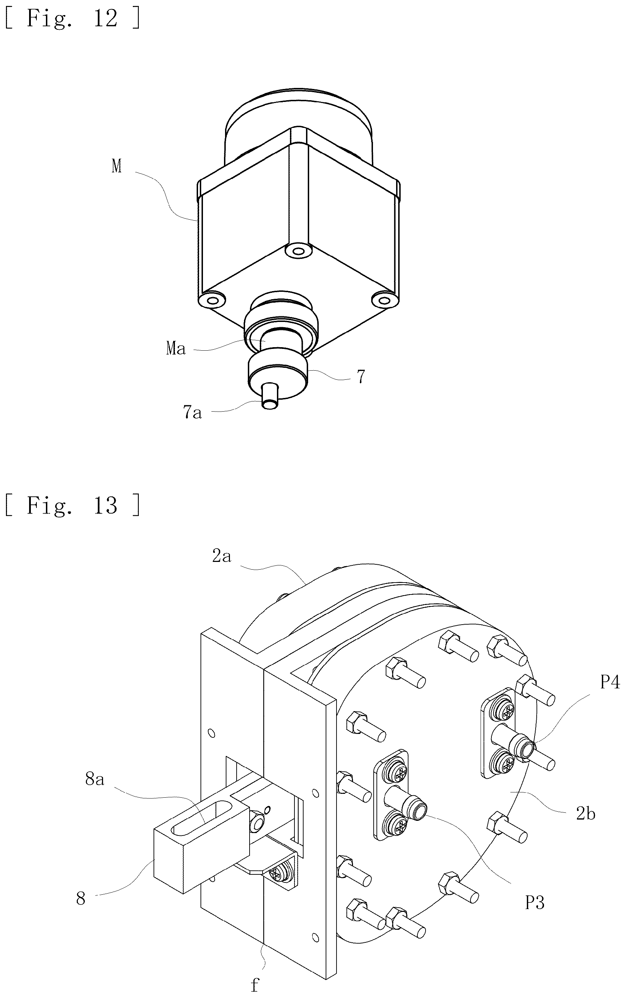

As illustrated in Figs. 7 and 11, the motor M includes an output shaft Ma, which is rotatable when activated. The output shaft Ma is provided at the distal end thereof with a cam member 7. As illustrated in Fig. 12, the cam member 7 has a protrusion 7a at a position thereof that is eccentric with respect to the direction of rotation. The cam member 7 is set with the protrusion 7a thereof received by the groove 8a of the cam-receiving member 8 that is coupled to the core member 6. As illustrated in Figs. 11 and 13, the groove 8a extends in the direction in which the extended portion 6b of the core member 6 is extended, and has a groove width that allows the insertion of the protrusion 7a.

Therefore, when the cam member 7 rotates together with the output shaft Ma of the motor M, the rotational force of the cam member 7 is received by the cam-receiving member 8 and is converted into a force that swings the core member 6 in the left-right direction. Specifically, when the motor M is activated, the rotational force of the motor M is transmitted through the cam member 7 and the cam-receiving member 8 to the core member 6 and causes the core member 6 to swing about the shaft member L. Thus, the dividing membrane 5 is displaceable into the first area 3 and into the second area 4 alternately.

Referring to Fig. 14, when the core member 6 swings with the activation of the motor M and displaces the dividing membrane 5 into the first area 3 (see Fig. 6), the dialysate in the first area 3 is discharged through the dialysate introduction line L1b into the blood purifier H, whereas the drain liquid in the blood purifier H is suctioned through the dialysate drain line L2b into the second area 4. Subsequently, when the core member 6 swings and displaces the dividing membrane 5 into the second area 4 (see Fig. 5), the dialysate in the second area 4 is discharged through the dialysate drain line L2a into the draining means, whereas the dialysate from the supply source is suctioned through the dialysate introduction line L1a into the first area 3.

According to the present embodiment, the dividing membrane 5 is provided and is displaced alternately into the first area 3 and into the second area 4 to cause the dialysate to be suctioned into and discharged from the first area 3 and cause the drain liquid to be suctioned into and discharged from the second area 4. Furthermore, the core member 6 is provided to the dividing membrane 5 and is configured to undergo a reciprocating motion inside the chamber 2 with the activation of the motor M (drive source) and in such a manner as to displace the dividing membrane 5 into the first area 3 and into the second area 4. Therefore, the chamber 2 is sealed in an easy and assured manner. Furthermore, the necessity for electromagnetic valves and/or additional pumps is eliminated.

Specifically, the dividing membrane 5 is displaceable into the first area 3 and into the second area 4 by the drive source that is the motor M, and the flows of the dialysate and the drain liquid are settable in desired directions with the use of the check valves V. Therefore, the dialysate introduction lines (L1a and L1b) and the dialysate drain lines (L2a and L2b) do not need to be provided with additional pumps and/or electromagnetic valves. Accordingly, no electric power for such additional pumps and/or electromagnetic valves is necessary.

The core member 6 according to the present embodiment is integrated into the dividing membrane 5. Therefore, the manufacturing cost is reduced. Furthermore, the dividing membrane 5 follows the core member 6 in an assured and favorable manner. Furthermore, the core member 6 is kept out of contact with the dialysate in the first area 3 and the drain liquid in the second area 4. The existing examples require a precise sealing structure for preventing liquid leakage at moving parts (sliding parts). To establish such a precise sealing structure, strict dimensional accuracy is required for relevant components, which increases the costs of the components. In contrast, according to the present embodiment, since the core member 6 is integrated into the dividing membrane 5, the above precise sealing structure is not necessary. That is, the dimensional accuracy required for the components is eased, which suppresses the manufacturing cost. Furthermore, the core member 6 undergoes a reciprocating motion inside the chamber 2 by swinging about the predetermined shaft member L in displacing the dividing membrane 5 inside the chamber 2. Therefore, compared with a case of a core member 6 that undergoes a sliding motion or the like in displacing the dividing membrane 5, the number of portions to be sealed is smaller, so that the sealing by the sealing portions 5b is ensured.

The chamber 2 has the fan-shaped accommodation space spreading along the locus of swing of the core member 6. Therefore, the dividing membrane 5 is favorably displaced in conformity with the reciprocating motion of the core member 6. Furthermore, the function of the dividing membrane 5 as a diaphragm is more smoothly and assuredly exerted. The dividing membrane 5 according to the present embodiment includes the dividing portion 5a and the sealing portions 5b that are formed integrally with each other. The dividing portion 5a separates the first area 3 and the second area 4 from each other. The sealing portions 5b seal the first area 3 and the second area 4. Therefore, while the function of the dividing membrane 5 as a diaphragm is exerted by the dividing portion 5a, a sealing function is maintained by the sealing portions 5b.

The drive source that activates the core member 6 is the motor M, and the rotational force generated by the motor M is transmitted through the cam member 7 to the core member 6 and causes the core member 6 to undergo a reciprocating motion. Since a general-purpose motor is employable as the drive source, the configuration is simplified while a necessary driving force is secured. The motor M may be replaced with another drive source such as a cylinder, and the cam member 7 may be replaced with another transmission means such as a link mechanism for the transmission of the driving force.

Now, a pump device according to another embodiment of the present invention will be described.

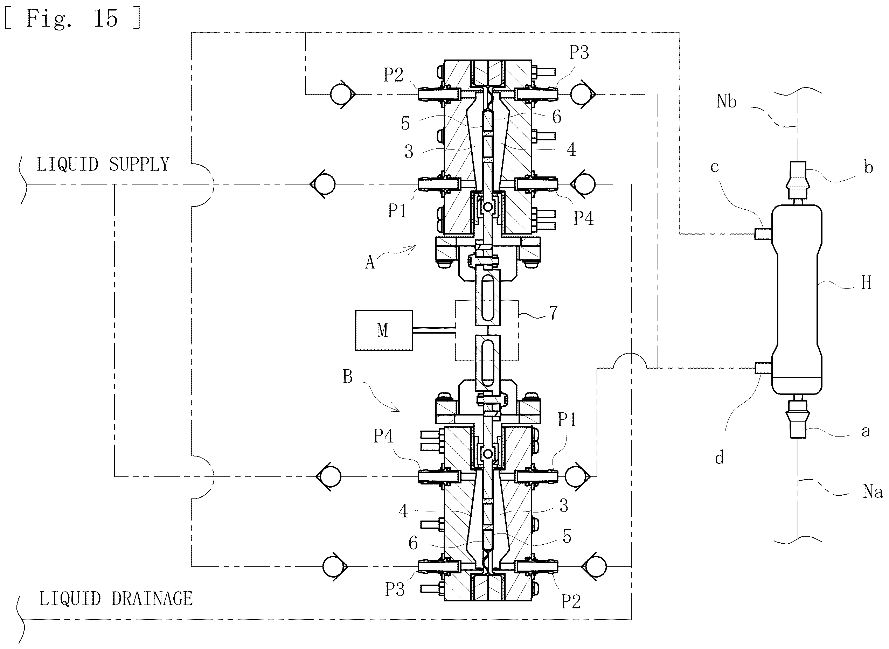

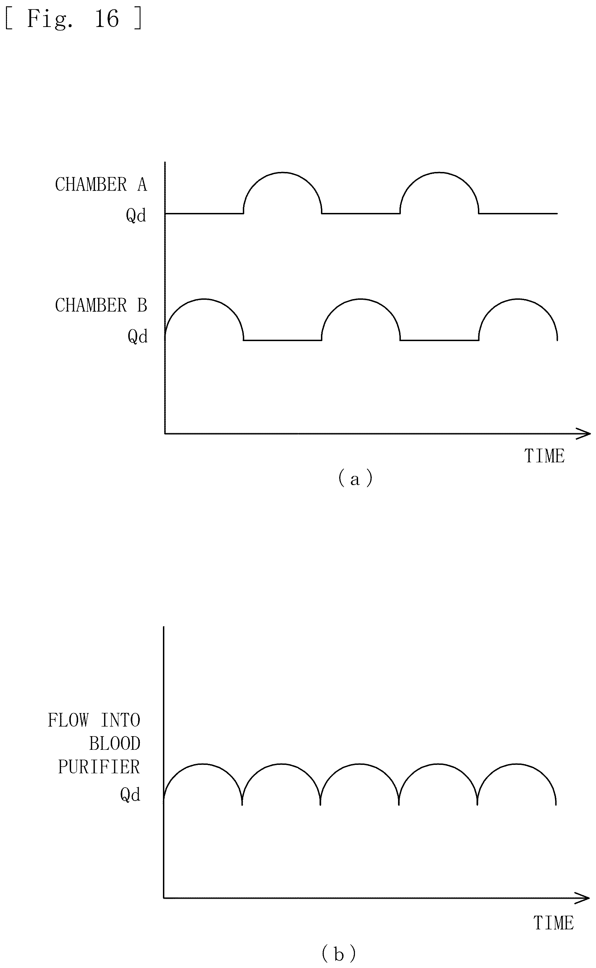

As illustrated in Fig. 15, the pump device according to the present embodiment includes a pair of chambers (one chamber A and an other chamber B) having respective dividing membranes 5. The chambers (A and B) have respective first areas 3 and respective second areas 4, which are connected to each other with flexible tubes or the like. Activating a motor M (drive source) causes the dividing membranes 5 of the respective chambers (A and B) to displace in opposite phases such that the suction and discharge of liquid is performed in a staggered manner between the chambers (A and B). In the drawing, the core member 6 of the one chamber A and the core member 6 of the other chamber B are connected to the motor M through a cam member 7. In the present embodiment, as illustrated in Fig. 16(a), dialysate is supplied from the one chamber A and from the other chamber B to the blood purifier H at respective flow rates Qd. Thus, as illustrated in Fig. 16(b), continuous supply of dialysate is enabled.

Now, a pump device according to yet another embodiment of the present invention will be described.

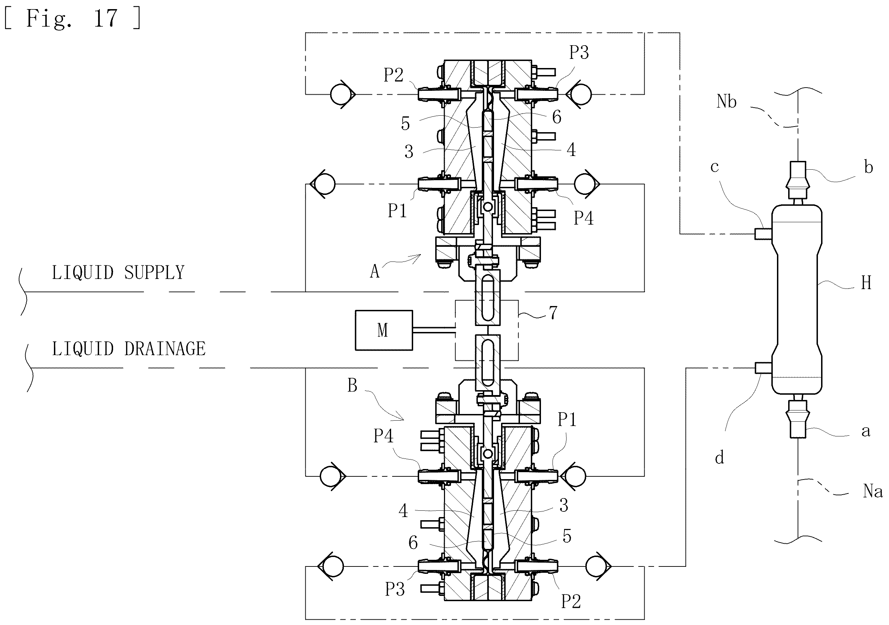

As illustrated in Fig. 17, the pump device according to the present embodiment includes a pair of chambers (one chamber A and an other chamber B) having respective dividing membranes 5. The chambers (A and B) have respective first areas 3 and respective second areas 4, which are connected to each other with flexible tubes or the like. Activating a motor M (drive source) causes the dividing membranes 5 of the respective chambers (A and B) to be displaced in opposite phases such that the one chamber A suctions the liquid and the other chamber B discharges the liquid. In the drawing, the core member 6 of the one chamber A and the core member 6 of the other chamber B are connected to the motor M through a cam member 7. In the present embodiment, the one chamber A is exclusively used for supplying the liquid, whereas the other chamber B is exclusively used for draining the liquid.

While the present embodiments have been described above, the present invention is not limited thereto. For example, instead of forming the core member 6 inside the dividing membrane 5 through insert molding so as to be integrated with the dividing membrane 5, the core member 6 may be simply inserted into the dividing membrane 5 to be integrated therewith. Furthermore, the core member 6 may swing in any direction, such as the top-bottom direction, other than the left-right direction. Moreover, instead of swinging, the core member 6 may move in another manner (a translational manner or the like) to undergo a reciprocating motion. The flow paths connected to the first connection ports (P1 and P2) and the second connection ports (P3 and P4) are not limited to the dialysate introduction lines (L1a and L1b) and the dialysate drain lines (L2a and L2b) according to the present embodiments and may be any flow paths that allow various kinds of liquid to flow through.

According to a first embodiment of the present invention, there is provided a pump device 1 including a chamber 2 capable of accommodating a predetermined volume of liquid; a dividing membrane 5 made of a flexible member attached to the chamber 2, the dividing membrane 5 dividing an inside of the chamber 2 into a first area 3 and a second area 4; and a core member 6 provided to the dividing membrane 5 and configured to undergo a reciprocating motion inside the chamber 2 with activation of a motor M (drive source) and in such a manner as to displace the dividing membrane 5 into the first area 3 and into the second area 4. Displacing the dividing membrane 5 alternately into the first area 3 and into the second area 4 causes the liquid to be suctioned into and discharged from the first area 3 and causes drain liquid to be suctioned into and discharged from the second area 4. Such a configuration produces the following effects: the chamber 2 is sealed in an easy and assured manner, the necessity for additional pumps is eliminated, and the flow rate of the liquid is controllable with high accuracy.

According to a second embodiment of the present invention, in the pump device 1 according to the first embodiment, the core member 6 is integrated into the dividing membrane 5. Such a configuration produces the following effects: the manufacturing cost is reduced, the dividing membrane 5 follows the core member 6 in an assured and favorable manner, and the core member 6 is kept out of contact with the dialysate in the first area 3 and the drain liquid in the second area 4.

According to a third embodiment of the present invention, in the pump device 1 according to the first embodiment, the core member 6 undergoes the reciprocating motion inside the chamber 2 by swinging about a predetermined shaft member L in displacing the dividing membrane 5 inside the chamber 2. Such a configuration produces the following effects: compared with a case of a core member 6 that undergoes a sliding motion or the like in displacing the dividing membrane 5, the number of portions to be sealed is smaller, so that the sealing by the sealing portions 5b is ensured.

According to a fourth embodiment of the present invention, in the pump device 1 according to the third embodiment, the chamber 2 has a fan-shaped accommodation space spreading along a locus of swing of the core member 6. Such a configuration produces the following effects: the dividing membrane 5 is favorably displaced in conformity with the reciprocating motion of the core member 6, and the function of the dividing membrane 5 as a diaphragm is more smoothly and assuredly exerted.

According to a fifth embodiment of the present invention, in the pump device 1 according to any one of the first to fourth embodiments, the dividing membrane 5 includes a dividing portion 5a and a sealing portion 5b that are formed integrally with each other, the dividing portion 5a separating the first area 3 and the second area 4 from each other, the sealing portion 5b sealing the first area 3 and the second area 4. Such a configuration produces the following effects: while the function of the dividing membrane 5 as a diaphragm is exerted by the dividing portion 5a, a sealing function is maintained by the sealing portion 5b.

According to a sixth embodiment of the present invention, in the pump device 1 according to the first embodiment, the drive source that activates the core member 6 is a motor M, and a rotational force generated by the motor M is transmitted through a cam member 7 to the core member 6 and causes the core member 6 to undergo the reciprocating motion. Such a configuration produces the following effects: since a general-purpose motor is employable as the drive source, the configuration is simplified while a necessary driving force is secured.

According to a seventh embodiment of the present invention, the pump device 1 according to the first embodiment further includes a valve configured to cause the liquid to be suctioned into and discharged from the first area 3 and cause the drain liquid to be suctioned into and discharged from the second area 4. Such a configuration enables an accurate control of the flow of the liquid into and from the first area 3 and the second area 4.

According to an eighth embodiment of the present invention, in the pump device 1 according to the first embodiment, a pair of chambers 2 each having the dividing membrane 5 is provided, the first areas 3 and the second areas 4 of the respective chambers 2 are connected to each other, and activating the drive source causes the dividing membranes 5 of the respective chambers 2 to be displaced in opposite phases such that the suction and discharge of the liquid is performed in a staggered manner between the chambers 2. Such a configuration produces the following effects: a continuous flow of the liquid is generated, and the flow rate of the liquid is controllable with higher accuracy.

According to a ninth embodiment of the present invention, in the pump device 1 according to the first embodiment, a pair of chambers 2 each having the dividing membrane is provided, the first areas 3 and the second areas 4 of the respective chambers 2 are connected to each other, and activating the drive source causes the dividing membranes 5 of the respective chambers 2 to be displaced in opposite phases such that one chamber A suctions the liquid and an other chamber B discharges the liquid. Such a configuration produces the following effects: the necessity for additional pumps is eliminated, and the flow rate of the liquid is controllable with high accuracy.

### Industrial Applicability

The present invention is also applicable to any pump device having a different appearance, additional functions, or the like within the spirit of the present invention.

### Reference Signs List

1 pump device
2 chamber
2a first chamber
2b second chamber
3 first area
4 second area
5 dividing membrane
5a dividing portion
5b sealing portion
6 core member
6a distal portion
6b extended portion
6ba insertion hole
6bb screw hole
7 cam member
7a protrusion
8 cam-receiving member
8a groove
M motor (drive source)
Ma output shaft
L shaft member
P1, P2 first connection port
P3, P4 second connection port
V check valve
H blood purifier

## Claims

1. A pump device comprising:
a chamber capable of accommodating a predetermined volume of liquid;
a dividing membrane made of a flexible member attached to the chamber, the dividing membrane dividing an inside of the chamber into a first area and a second area; and
a core member provided to the dividing membrane and configured to undergo a reciprocating motion inside the chamber with activation of a drive source and in such a manner as to displace the dividing membrane into the first area and into the second area,
wherein displacing the dividing membrane alternately into the first area and into the second area causes the liquid to be suctioned into and discharged from the first area and causes drain liquid to be suctioned into and discharged from the second area.

2. The pump device according to claim 1, wherein the core member is integrated into the dividing membrane.

3. The pump device according to claim 1, wherein the core member undergoes the reciprocating motion inside the chamber by swinging about a predetermined shaft member in displacing the dividing membrane inside the chamber.

4. The pump device according to claim 3, wherein the chamber has a fan-shaped accommodation space spreading along a locus of swing of the core member.

5. The pump device according to any one of claims 1 to 4, wherein the dividing membrane includes a dividing portion and a sealing portion that are formed integrally with each other, the dividing portion separating the first area and the second area from each other, the sealing portion sealing the first area and the second area.

6. The pump device according to claim 1, wherein the drive source is a motor, and wherein a rotational force generated by the motor is transmitted through a cam member to the core member and causes the core member to undergo the reciprocating motion.

7. The pump device according to claim 1, further comprising a valve configured to cause the liquid to be suctioned into and discharged from the first area and cause the drain liquid to be suctioned into and discharged from the second area.

8. The pump device according to claim 1, wherein a pair of chambers each having the dividing membrane is provided, wherein the first areas and the second areas of the respective chambers are connected to each other, and wherein activating the drive source causes the dividing membranes of the respective chambers to be displaced in opposite phases such that the suction and discharge of the liquid is performed in a staggered manner between the chambers.

9. The pump device according to claim 1, wherein a pair of chambers each having the dividing membrane is provided, wherein the first areas and the second areas of the respective chambers are connected to each other, and wherein activating the drive source causes the dividing membranes of the respective chambers to be displaced in opposite phases such that one of the chambers suctions the liquid and an other of the chambers discharges the liquid.
